# EUROPEAN PATENT SPECIFICATION

(11) **EP 3 845 201 B1**
(45) Date of publication and mention of the grant of the patent: **14.10.2026**
(21) Application number: 20217399.3
(22) Date of filing: 28.12.2020
(51) Int. Cl.: A61C 19/05

(54) **OCCLUSAL PRESSURE ANALYSIS PROGRAM**
PROGRAMM ZUR ANALYSE DES AUFBISSDRUCKS
PROGRAMME D'ANALYSE DE LA PRESSION OCCLUSALE

(30) Priority: 06.01.2020 JP 2020000370
(43) Date of publication of application: 07.07.2021
(73) Proprietor: The Nippon Dental University, Chiyoda-ku Tokyo 102-8159 (JP); GC Corporation, Sunto-gun, Shizuoka 410-1307 (JP)
(72) Inventor: SHIGA, Hiroshi, Chiyoda-ku, Tokyo 102-8159 (JP); SHIRAISHI, Tomohisa, Itabashi-ku, Tokyo 174-8585 (JP); NOGUCHI, Yukie, Itabashi-ku, Tokyo 174-8585 (JP)
(74) Representative: Müller-Boré & Partner Patentanwälte PartG mbB

(56) References cited:
- EP-A1- 2 564 809
- EP-A1- 3 097 888
- JP-A- 2015 136 620
- US-A1- 2019 060 034

## Description

### FIELD

The present invention relates to programs for analyzing occlusal pressure in an occlusal contact state of upper and lower tooth rows.

### BACKGROUND

Pressure sensitive films (pressure sensitive sheets) and sensor sheets are known as means for measuring occlusal pressure (for example, Patent Literatures 1 to 4). When a subject bites a pressure sensitive film, the pressure sensitive film is strongly pressed at an occlusal contact portion of the upper and lower tooth rows, and a visual or electrical change appears. Depending on the type of the pressure sensitive film, differences in shade or color may appear depending on the magnitude of the occlusal pressure (contact pressure). In this manner, the occlusal pressure of the upper and lower tooth rows can be known with the pressure sensitive film.

### Citation List

### Patent Literature

Patent Literature 1: JP H06-213738 A
Patent Literature 2: JP H06-189980 A
Patent Literature 3: JP H06-180260 A
Patent Literature 4: JP 2005-279094 A

Documents JP2015136620A and EP3097888A1 describe a sensor sheet for detecting occlusal force by providing an X-electrode sheet and a Y-electrode sheet provided with a plurality of electrode wires on both faces of an organic piezoelectric film. An information processing device performs integration operation processing in each intersection point to address information of each intersection point output by an occlusal force detection device having the sensor sheet using an organic piezoelectric film, and raw data including data based on a signal showing a change in occlusal force and creates data of the occlusal force.

### SUMMARY

### Technical Problem

Pressure sensitive films show variations corresponding to the occlusal pressure at the site where the occlusal contact has occurred. This shows visual changes at the occlusal contact position, and the occlusal position and the pressure can be identified. However, since tooth surfaces have three-dimensional shapes with complicated irregularities, the pressure sensitive film experiences slippage while contacting the upper and lower teeth between the time when the subject starts to bite the pressure sensitive film and the time when the subject reaches the final occlusal position, and the process of this slippage also appears as a visual change in the pressure sensitive film. The influence of complicated folding of the sheet and the influence of adhering matters at the time of analysis of the occlusal pressure also may appear.

Such variations and influences in the pressure sensitive film have sometimes disturbed accurate grasp of the occlusal contact state. That is, when the occlusal pressure is acquired with the pressure sensitive film, the occlusal pressure different from the occlusal pressure at the final occlusal state is detected in the occlusion process or the analysis process, which have sometimes disturbed accurate grasp of the occlusal contact state. This is not limited to pressure sensitive films, and the same thing happens with the means for electrically obtaining the occlusal position and the occlusal pressure.

An object of the present invention is to provide an occlusal pressure analysis program capable of improving the accuracy of grasping the occlusal contact state when an occlusal pressure is obtained from a pressure sensitive means.

### Solution to Problem

The claimed invention is defined by the features set forth in the appended independent claim. An additional embodiment of the claimed invention is defined by the dependent claim.

### Advantageous Effects of Invention

According to the present invention, it is possible to improve the accuracy of grasping the occlusal contact state in obtaining the occlusal pressure by the pressure sensitive means.

### BRIEF DESCRIPTION OF DRAWINGS

Fig. 1 is a diagram showing a flow of an occlusal pressure analysis program S10.
Fig. 2 is a diagram to explain detection units P and detection groups G.
Fig. 3 is a diagram schematically showing a distribution of the detection groups G.
Fig. 4 is a diagram to explain a structure of an electronic computer 10.

### DETAILED DESCRIPTION OF EMBODIMENTS

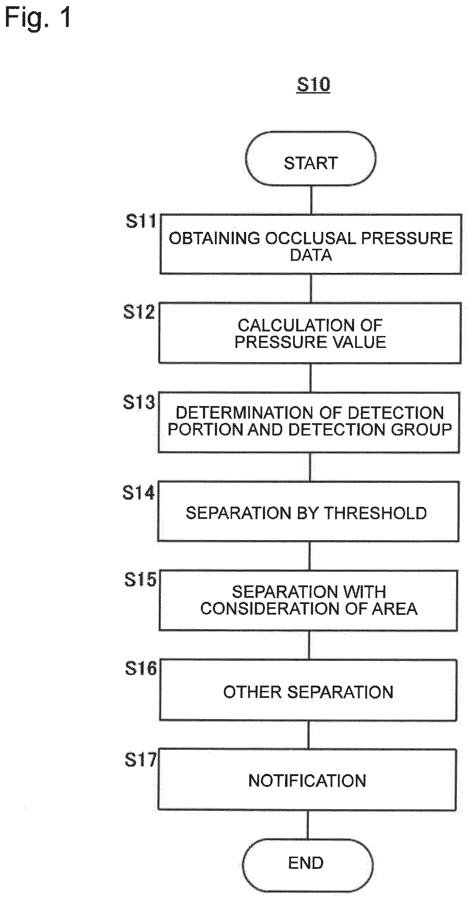

Fig. 1 is a diagram showing a flow of an occlusal pressure analysis program S10 according to one embodiment. As can be seen from Fig. 1, the occlusal pressure analysis program S10 includes steps S11 to S17. Hereinafter, each step will be described.

### <Step S11 of Obtaining Occlusal Pressure Data>

In the step S11 of obtaining occlusal pressure data (it may be referred to as "step S11"), positional information and detection information are obtained with respect to a portion where a predetermined information is detected by the pressure sensitive means (in the case of a pressure sensitive film, the portion where a visual change is made). That is, the data of the two-dimensional positional information (e.g., X, Y) of the detection distribution of the pressure sensitive means including the occlusal pressure distribution information and the detection information (C_{XY}) are obtained.

Here, the pressure sensitive means is not particularly limited, and pressure sensitive films (pressure sensitive sheets), pressure sensitive sensors that detect the occlusal contact position by variations in electrical characteristic, and the like may be exemplified. Among them, pressure sensitive films are preferable from the viewpoint of higher versatility and easy display of occlusal pressure.

The method of obtaining the data of positional information and detection information from the pressure sensitive means is not particularly limited. For example, if the pressure sensitive means is a pressure sensitive film, scanners and cameras for reading color or shade appeared on the pressure sensitive film may be used. If the pressure sensitive means is a pressure sensitive sensor, devices for measuring electrical features (voltage, current, resistance value, and changes in these features) may be exemplified.

As a more specific example, for each minimum unit (for example, one pixel), the coordinates thereof may be used as the positional information, and the color (RGB value) may be used as the detection information.

### <Step S12 of Calculating Pressure Value>

In the step S12 of calculating pressure value (it may be referred to as "step S12"), the detection information obtained in the step S11 is converted into a pressure value. That is, for each minimum unit, the obtained detection information (RGB value in the case of a pressure sensitive film) is converted into a pressure value by a relational expression obtained in advance. This provides a pressure value for each minimum unit.

### <Step S13 of Determining Detection Unit and Detection Group>

In the step S13 of determining detection units and detection groups (it may be referred to as "step S13"), a position where the pressure value is obtained and a position where the pressure value is not obtained are sorted out from the pressure value obtained in step S12 (identification of the detection units), and for a portion in which a plurality of detection units form a continuous one mass, its area is obtained as one detection group, and a characteristic value based on the pressure is calculated.

From this, a plurality of detection groups and detection units that does not belong to the detection groups are determined, and for each of them, the characteristic value based on the area and the pressure is obtained.

More specifically, for example, the following processes are performed.

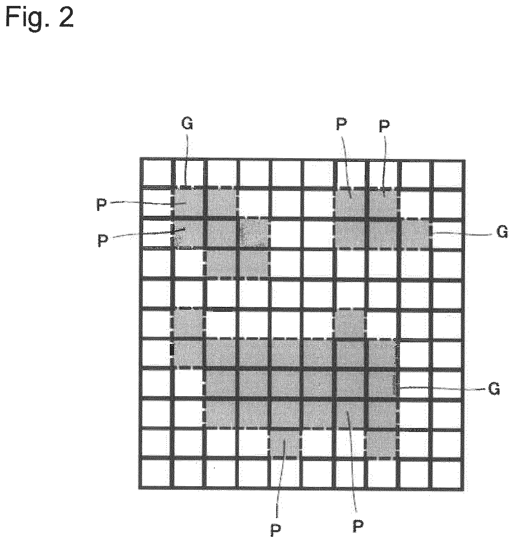

As shown in Fig. 2, the minimum unit where the pressure value is obtained (the pressure is not 0) for each minimum unit (pixel in this embodiment) is used as the detection unit. In Fig. 2, each pixel filled with thin gray is a detection unit P. When some of the detection units among the plurality of detection units are adjacent to each other and exist continuously, the detection units adjacent to each other are considered as one detection group. Each of the areas surrounded by dashed lines in Fig. 2 is a detection group G.

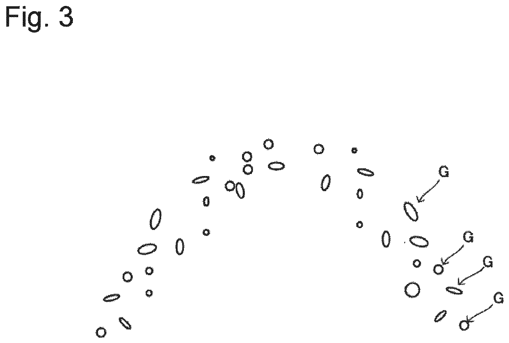

Specifically, a plurality of detection units, which exist continuously and form a group, are defined as the detection group by labeling. In this embodiment, the 8-connectivity processing is used to make the detection units continuous in the vertical, horizontal, and diagonal directions into the same label. This makes it possible to obtain distributions of the detection groups having different areas, as schematically shown in Fig. 3.

For each detection group, the area of the detection group is calculated from the number of the detection units belonging to the detection group, and the pressure characteristic value is calculated based on each pressure value of the detection units belonging to the detection group. This pressure characteristic value is a pressure value to characterize the pressure state of the detection group and to treat the detection group as one pressure state. Specifically, for example, the average value of the pressures of the detection units belonging to the detection group can be set as the pressure characteristic value, or the maximum value or the minimum value of the pressure value of the detection units belonging to the detection group can be set as the pressure characteristic value.

For a detection unit that does not belong to any detection groups and exists independently of other detection units, its pressure value is used as the pressure characteristic value.

### <Separation Step S14 with Threshold>

In the step S14 (it may be referred to as "step S14"), the detection groups and the detection units (not belonging to any detection groups) are separated based on the pressure characteristic value into the ones that can be regarded as detection due to occlusion and the ones that are determined to be unclear as to whether the detection is due to occlusion.

If the pressure characteristic value is equal to or larger than the predetermined pressure characteristic value, the detection groups and the detection units (not belonging to any detection groups) can be determined that they can be regarded as due to occlusion and not due to noise without taking other matters into consideration. Therefore, the detection groups and the detection unis (not belonging to any detection groups) whose pressure characteristic value is equal to or larger than the predetermined pressure characteristic value are regarded as due to occlusion. Specifically, for example, the determination includes "the detection groups and the detection units (not belonging to any detection groups) whose pressure characteristic value is equal to or greater than P₁ [MPa] are the detection unit due to occlusion (P₁ is a specific numerical value)".

### <Separation Step S15 with Consideration of Area>

In the separation step S15 (it may be referred to as "step S15") with the consideration of the area, the detection groups and the detection units (not belonging to any detection groups) which are determined to be due to occlusion in the step S14 (not belonging to any detection groups) are further separated into the detection groups and the detection units (not belonging to any detection groups) which are determined to be due to occlusion and the detection groups and the detection units (not belonging to any detection groups) which are unclear whether they are due to occlusion, based on the threshold of the pressure characteristic value associated with the area.

In step S14, even among the detection groups and the detection units (not belonging to any detection groups) which are unclear whether the detection is due to occlusion, the separation is made into the detection groups and the detection units (not belonging to any detection groups) which are considered to be based on occlusal contact and the detection groups and the detection units which are unclear whether the detection is due to occlusion, from the data base in which the relationship between the area of the occlusal contact portion and the occlusal pressure thereof has been searched in advance.

With this selection, the occlusal contact state can be represented more accurately.

According to the claimed invention, with respect to the detection groups and the detection units (not belonging to any detection groups) which are determined in S14 as unclear whether the detection is due to occlusion, if the area is equal to or larger than A₁ mm² and smaller than A₂ mm², it is determined that it is all unclear whether the detection is due to occlusion (for example, making P₁ [MPa] as the upper limit of the threshold, all the detection groups and the detection units whose pressure characteristic value is less than P₁ [MPa]), and with respect to the detection groups and the detection units (not belonging to any detection groups) whose area is equal to or larger than A₂ mm², making P₂ [MPa] as the upper limit of the threshold, the detection groups and the detection units (not belonging to any detection groups) having a pressure characteristic value higher than P₂ [MPa] are considered to be due to occlusion. Here, each of A₁, A₂, P₂ is a specific number (P₁ is the same as above). However, A₁ < A₂ and P₁ > P₂. Specific numerical values entering there can be determined from the characteristics of the pressure sensitive means (pressure sensitive sheet, etc.), and the values are obtained in advance depending on the pressure sensitive means to be used. The method for obtaining the specific numerical values is not particularly limited, and if the pressure sensitive means is a pressure sensitive sheet, it is possible, for example, to intentionally generate the detection units and the detection groups by giving an assumed bending or slippage to the pressure sensitive sheet, and the value generated by this may be used.

The relationship between the areas of the detection groups and the detection units not belonging to any detection groups and the threshold of the upper limit of the pressure characteristic value is not particularly limited, and the upper limit of the pressure characteristic value at the threshold can be made to be smaller as the areas of the detection group and the detection unit not belonging to any detection groups is larger. This can further improve the accuracy.

### <Other Separation Step S16>

In addition to the above, in the other separation step S16 (it may be referred to as "step S16"), when the detection group is a line segment, a separation may be carried out at least by one of: the detection groups and the detection units (not belonging to any detection groups) which are clearly separated from the adjacent detection groups or the detection units (not belonging to any detection groups); and the color of the detection groups and the detection units not belonging to any detection groups.

With respect to the color of the detection groups and the detection units not belonging to any of the detection groups, for example, it is possible to form a database in advance based on R value, G value, and B value in associate with that the detection is not due to occlusion when the color does not fall in a predetermined range. However, this step S16 is not necessarily provided, and may be provided as necessary.

### <Notification Step S17>

In the notification step S17 (it may be referred to as "step S17"), the detection groups and the detection units (not belonging to any detection groups) regarded as due to occlusion by the various separations in the steps S14 to S16, and the ones whose detection is unclear whether it is due to occlusion, are notified differently. The method of notification is not particularly limited, and may be performed by, for example, color, brightness, and both on a screen.

How to treat the detection groups and the detection units (not belonging to any detection groups) which are notified in the step S17 and whose detection is unclear whether it is due to occlusion may be appropriately set as necessary. For example, it may be specified and deleted one by one, or to some extent collectively, all notified detection groups and detection units (not belonging to any detection groups) may be specified collectively and automatically deleted, and may be manually changed to detection groups and detection units (not belonging to any detection groups) due to occlusion.

According to the occlusal pressure analysis program S10 of the present embodiment, in obtaining the occlusal pressure by the pressure sensitive means, it is possible to separate a portion due to occlusion and a portion not due to occlusion accurately and efficiently, and it is possible to improve the accuracy of grasping the occlusal contact state.

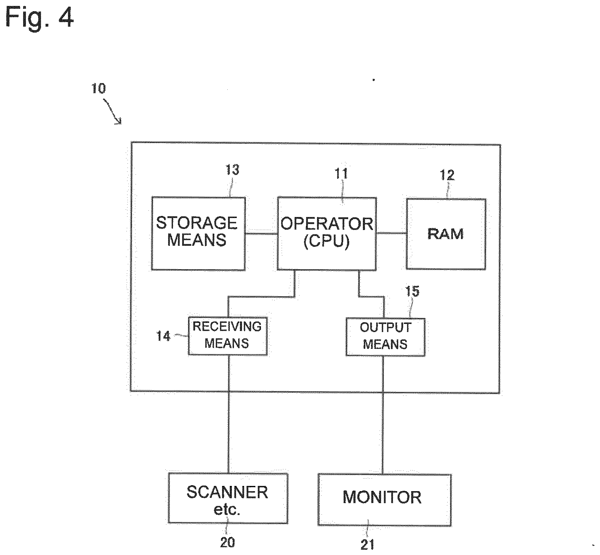

The occlusal pressure analysis program S10 to which these steps belong is performed by an electronic computer such as a computer. That is, the occlusal pressure analysis program S10 including the steps S11 to S17 is performed by an electronic computer performing an operation and outputting the results. Fig. 4 shows a diagram for explanation. As shown in Fig. 4, an electronic computer 10 includes an operator 11, a RAM 12, a storage means 13, a receiving means 14, and an outputting means 15.

The operator 11 is composed of a so-called CPU (Central Processing Unit), and is connected to the above-mentioned structural members and is a means capable of controlling them. The operator 11 also executes various programs stored in the storage means 13 or the like that functions as a storage medium, and performs an operation as a means for generating various data and selecting data based on the programs. In this embodiment, the storage means 13 stores the occlusal pressure analysis program S10 and the operator 11 operates the program to obtain the results.

RAM 12 is a member that functions as a working area of the operator 11 and a temporary data-storage means. RAM 12 may be composed of a SRAM, DRAM, a flash memory, or the like, and is similar to known RAMs.

The storage means 13 is a member that functions as a storage medium for storing programs and data as a basis for various operations. The storage means 13 may also be capable of storing intermediate and final results obtained by the execution of the program. In this embodiment, the occlusal pressure analysis program S10 is stored in the storage means 13.

The receiving means 14 is connected to a scanner 20 for example, and is a member having a function to incorporate image information based on the pressure sensitive means from the scanner.

The output means 15 is a member having a function to output information to be output to the outside among the results obtained by the operation of the operator 11, and in this embodiment, is connected to the monitor 21, from which the person who operates the electronic computer can see the result on the screen.

According to the electronic computer 10, the data presented on the pressure sensitive means from a reading medium such as a scanner is taken into the electronic computer 10 via the receiving means 14, the operator 11 performs an operation based on the occlusal pressure analysis program S10 stored in the storage means 13, and the results are displayed on the monitor 21 via the output means 15.

This makes it possible for the person who operates the computer to obtain the occlusal contact state in which data of an unnecessary portion is finally deleted and accuracy is enhanced.

### References Sign List

- 10: electronic computer
- 11: operator
- 12: RAM
- 13: storage means
- 14: receiving means
- 15: output means

## Claims

1. An occlusal pressure analysis program for obtaining an occlusal pressure distribution from a pressure sensitive means, the occlusal pressure analysis program comprising:
a step (S12) of obtaining a pressure value for each minimum unit from which the pressure value can be recognized by the pressure sensitive means;
a step (S13) of obtaining areas and pressure characteristic values of detection groups (G), regarding the minimum unit where the pressure value exists as a detection unit (P) and regarding a group of a plurality of the detection units (P) that are existing continuously as each of the detection groups (G);
a step (S14) of regarding a detection group (G) whose pressure characteristic value is equal to or greater than a predetermined pressure (P₁) as a detection group (G) due to occlusion; and
a step (S15) of regarding the detection group (G) as unclear whether the detection group (G) is due to occlusion, if the area of the detection group (G) is equal to or larger than a first predetermined area (A₁) and smaller than a second predetermined area (A₂) and the pressure characteristic value of the detection group (G) is smaller than a first predetermined pressure (P₁) as an upper limit of a threshold, and regarding the detection group (G) whose area is equal to or larger than the second predetermined area (A₂) and having a pressure characteristic value higher than a second predetermined pressure (P₂) as the upper limit of the threshold, as due to occlusion, wherein the first predetermined area (A₁) is lower than the second predetermined area (A₂) and the first predetermined pressure (P₁) is higher than the second predetermined pressure (P₂).

2. The occlusal pressure analysis program according to claim 1 , further comprising a step of separating the detection groups (G) into the detection groups (G) which are due to occlusion and the detection groups (G) which are unclear whether they are due to occlusion, from at least one of the shape, distance from the other detection groups (G), and color of the detection group (G).

## Patentansprüche

1. Ein Programm zur Analyse des okklusalen Drucks zum Erhalten einer Verteilung des okklusalen Drucks anhand eines drucksensitiven Mittels, wobei das Programm zur Analyse des okklusalen Drucks Folgendes umfasst:
einen Schritt (S12) zum Erhalten eines Druckwerts für jede Minimaleinheit, von der der Druckwert durch das drucksensitive Mittel erkannt werden kann;
einen Schritt (S13) zum Erhalten von Flächen und Druckkennwerten von Detektionsgruppen (G), wobei die Minimaleinheit, in der der Druckwert vorliegt, als Detektionseinheit (P) angesehen wird, und wobei eine Gruppe aus einer Vielzahl der Detektionseinheiten (P), die kontinuierlich vorliegen, als jede der Detektionsgruppen (G) angesehen wird;
einen Schritt (S14) des Ansehens einer Detektionsgruppe (G), deren Druckkennwert gleich oder größer als ein vorbestimmter Druck (P1) ist, als eine auf Okklusion zurückzuführende Detektionsgruppe (G); und
einen Schritt (S15) des Ansehens der Detektionsgruppe (G) als unklar dahingehend, ob sie auf Okklusion zurückzuführen ist, wenn die Fläche der Detektionsgruppe (G) gleich oder größer als eine erste vorbestimmte Fläche (A₁) und kleiner als eine zweite vorbestimmte Fläche (A₂) ist, und der Druckkennwert der Detektionsgruppe (G) kleiner als ein erster vorbestimmter Druck (P₁) als Obergrenze eines Schwellenwerts ist, und des Ansehens der Detektionsgruppe (G), deren Fläche gleich oder größer als die zweite vorbestimmte Fläche (A₂) ist, und die einen Druckkennwert aufweist, der höher ist als ein zweiter vorbestimmter Druck (P₂) als Obergrenze des Schwellenwerts, als auf eine Okklusion zurückzuführen, wobei die erste vorbestimmte Fläche (A₁) kleiner ist als die zweite vorbestimmte Fläche (A₂) und der erste vorbestimmte Druck (P₁) höher ist als der zweite vorbestimmte Druck (P₂).

2. Das Programm zur Analyse des okklusalen Drucks nach Anspruch 1, ferner umfassend einen Schritt des Trennens der Detektionsgruppen (G) in die Detektionsgruppen (G), die auf eine Okklusion zurückzuführen sind, und in die Detektionsgruppen (G), bei denen unklar ist, ob sie auf eine Okklusion zurückzuführen sind, anhand mindestens eines von der Form, dem Abstand von den anderen Detektionsgruppen (G) und der Farbe der Detektionsgruppe (G).

## Revendications

1. Un programme d'analyse de la pression occlusale destiné à obtenir une distribution de la pression occlusale à partir d'un moyen sensible à la pression, ledit programme d'analyse de la pression occlusale comprenant :
une étape (S12) consistant à obtenir une valeur de pression pour chaque unité minimale à partir de laquelle la valeur de pression peut être détectée par le moyen sensible à la pression ;
une étape (S13) consistant à obtenir des surfaces et des valeurs caractéristiques de pression de groupes de détection (G), l'unité minimale au niveau de laquelle la valeur de pression existe étant considérée comme une unité de détection (P), et un groupe constitué d'une pluralité d'unités de détection (P) qui existent de manière continue étant considéré comme chacun des groupes de détection (G) ;
une étape (S14) consistant à considérer un groupe de détection (G) dont la valeur caractéristique de pression est égale ou supérieure à une pression prédéterminée (P₁) comme un groupe de détection (G) dû à une occlusion ; et
une étape (S15) consistant à considérer le groupe de détection (G) comme incertain quant à savoir si ce groupe de détection (G) est dû à une occlusion, si la surface du groupe de détection (G) est égale ou supérieure à une première surface prédéterminée (A₁) et inférieure à une deuxième surface prédéterminée (A₂), et si la valeur caractéristique de pression du groupe de détection (G) est inférieure à une première pression prédéterminée (P₁) servant de limite supérieure d'un seuil, et à considérer que le groupe de détection (G) dont la surface est égale ou supérieure à la deuxième surface prédéterminée (A₂) et présentant une valeur caractéristique de pression supérieure à une deuxième pression prédéterminée (P₂) servant de limite supérieure du seuil, est dû à une occlusion, sachant que la première surface prédéterminée (A₁) est inférieure à la deuxième surface prédéterminée (A₂) et que la première pression prédéterminée (P₁) est supérieure à la deuxième pression prédéterminée (P₂).

2. Le programme d'analyse de la pression occlusale d'après la revendication 1, comprenant en outre une étape consistant à séparer les groupes de détection (G) en groupes de détection (G) dus à une occlusion et en groupes de détection (G) pour lesquels il n'est pas certain qu'ils soient dus à une occlusion, sur la base d'au moins l'un parmi : la forme, la distance par rapport aux autres groupes de détection (G) et la couleur du groupe de détection (G).
